# DEMANDE DE BREVET EUROPEEN

(11) **EP 1 022 271 A1**
(43) Date de publication de la demande: **26.07.2000**
(21) Numéro de dépôt: 99403216.7
(22) Date de dépôt: 20.12.1999
(51) Int. Cl.: C07D 233/61, C07D 295/12, C07C 211/63, A61K 7/13

(54) **Nouveaux colorants mono-benzéniques cationiques, leur utilisation pour la teinture d'oxydation des fibres kératiniques, compositions tinctoriales et procédés de teinture**

(30) Priorité: 19.01.1999 FR 9900504
(71) Demandeur: L'OREAL, 75008 Paris (FR)
(72) Inventeur: Genet, Alain, 93600 Aulnay-sous-Bois (FR); Lagrange Alain, 77700 Coupvray (FR)
(74) Mandataire: Goulard, Sophie

(57) **Abrégé**

L'invention a pour objet de nouveaux colorants mono-benzéniques comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, leur utilisation à titre de précurseur de colorants d'oxydation pour la teinture d'oxydation des fibres kératiniques, les compositions tinctoriales les contenant, ainsi que les procédés de teinture d'oxydation les mettant en oeuvre.

## Description

L'invention a pour objet de nouveaux colorants mono-benzéniques comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, leur utilisation à titre de précurseur de colorants d'oxydation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que par exemples des coupleurs indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir, de façon totalement inattendue et surprenante, que de nouveaux colorants mono-benzéniques cationiques de formule (I) ci-après définie comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quaternisé, non seulement conviennent pour une utilisation comme précurseurs de colorants d'oxydation, mais en outre qu'ils permettent d'obtenir des compositions tinctoriales conduisant à des colorations puissantes, dans une large palette de couleurs, et présentant d'excellentes propriétés de résistances aux différents traitements que peuvent subir les fibres kératiniques.

Ces découvertes sont à la base de la présente invention.

L'invention a donc pour premier objet de nouveaux composés de formule (I) suivante, et leurs sels d'addition avec un acide : 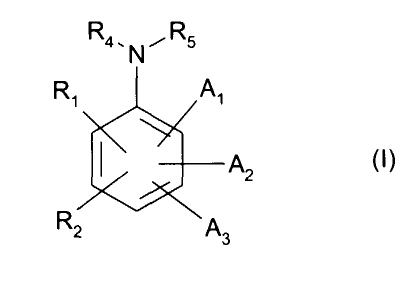 dans laquelle :
- A₁, A₂ et A₃, identiques ou différents, représentent un groupement -NR₇R₈ ou un radical hydroxyle ; un et un seul des groupements A₁, A₂ et A₃ peut en outre représenter un groupement R3 tel que défini ci-après ;
   ● • R₁, R₂ et R₃, qui peuvent être identiques ou différents, représentent un atome d'hydrogène ; un atome d'halogène ; un groupement Z tel que défini ci-après ; un radical alkyl(C₁-C₆) carbonyle ; un radical aminoalkyl(C₁-C₆)carbonyle; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle; un radical aminoalkyl(C₁- C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆); un radical N,N-dialkyl(C₁-C₆)aminosulfonylalkyle(C₁-C₆) ; un radical carbamyle ; un radical N-alkyl(C₁-C₆)carbamyle; un radical N,N-dialkyl(C₁-C₆)carbamyle; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆) carboxy, carbamyle, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, ou par un groupement Z tel que défini ci-après dans lequel le bras de liaison D contient une contient une fonction cétone directement reliée à l'atome d'azote dudit groupe amino ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle, N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z tels définis ci-après, ou pouvant former ensemble, avec l'atome d'azote auquel ils sont rattachés, un cycle à 5 ou 6 chaînons, carboné, ou contenant un ou plusieurs hétéroatomes ;
   ● • R₆ désigne un radical alkyle en C₁-C₆ ; un radical monohydroxyalkyle en C₁-C₆ ; un radical polyhydroxyalkyle en C₂-C₆ ; un groupement Z tel que défini ci-après ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou parmi les groupements Z tels que définis ci-après ; ou pouvant former ensemble, avec l'atome d'azote auquel ils sont rattachés, un cycle à 5 ou 6 chaînons, carboné, ou contenant un ou plusieurs hétéroatomes ;
- R₄, R₅, R₇ et R₈, identiques ou différents, représentent un atome d'hydrogène ; un groupement Z tel que défini ci-après dans lequel le bras de liaison D ne contient pas de fonction cétone directement reliée à l'atome d'azote portant les radicaux R₄ et R₅ ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle, N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z tels que définis ci-après ; ou pouvant former ensemble, avec l'atome d'azote auquel ils sont rattachés, un cycle à 5 ou 6 chaînons, carboné, ou contenant un ou plusieurs hétéroatomes ;
- Z est choisi parmi les groupements cationiques insaturés de formules (II) et (III) suivantes, et les groupements cationiques saturés de formule (IV) suivante : 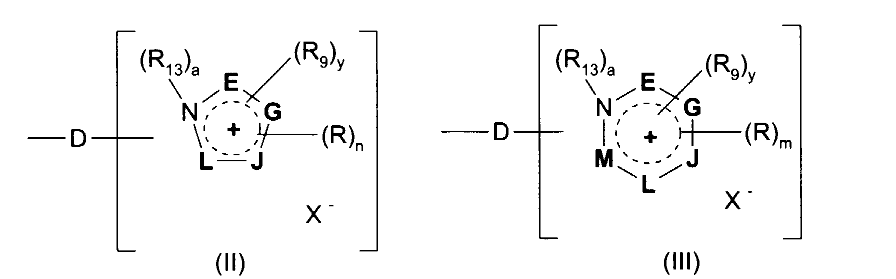 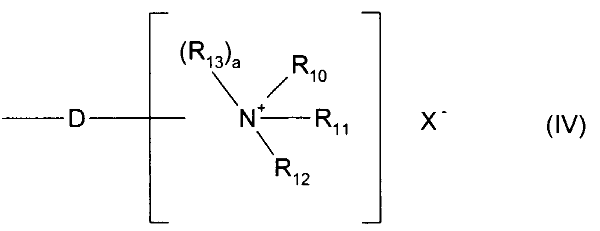 dans lesquelles :
- D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
- les sommets E, G, J, L et M, identiques ou différents, représentent un atome de carbone, d'oxygène, de soufre ou d'azote ;
- n est un nombre entier compris entre 0 et 4 inclusivement ;
- m est un nombre entier compris entre 0 et 5 inclusivement ;
- les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₉ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un second groupement Z identique ou différent du premier groupement Z ;
- R₁₀, R₁₁ et R₁₂, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₁₀, R₁₁ et R₁₂ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₁₀, R₁₁ et R₁₂ peut également représenter un second groupement Z, identique ou différent du premier groupement Z ;
- R₁₃ représente un radical alkyle en C₁-C₆ ; un radical monohydroxyalkyle en C₁-C₆ ; un radical polyhydroxyalkyle en C₂-C₆ un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- a et y sont des nombres entiers égaux à 0 ou 1 ; avec les conditions suivantes :
   - dans les groupements cationiques insaturés de formule (II);
   - lorsque a = 0, le bras de liaison D est rattaché à l'atome d'azote,
   - lorsque a = 1, le bras de liaison D est rattaché à l'un des sommets E, G, J ou L, - y ne peut prendre la valeur 1 que :
      1) lorsque les sommets E, G, J et L représentent simultanément un atome de carbone, et que le radical R₉ est porté par l'atome d'azote du cycle insaturé ; ou bien
      2) lorsqu'au moins un des sommets E, G, J et L représente un atome d'azote sur lequel le radical R₉ est fixé ;
         - dans les groupements cationiques insaturés de formule (III):
         - lorsque a = 0, le bras de liaison D est rattaché à l'atome d'azote,
         - lorsque a = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₉ est porté par l'atome d'azote du cycle insaturé ;
         - dans les groupements cationiques de formule (IV) :
         - lorsque a = 0, alors le bras de liaison D est rattaché à l'atome d'azote portant les radicaux R₁₀ à R₁₂,
         - lorsque a = 1, alors deux des radicaux R₁₀ à R₁₂ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
- X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
étant entendu que
- le nombre de groupement cationique Z est au moins égal à 1.

Comme indiqué précédemment, les colorations obtenues avec la composition de teinture d'oxydation contenant le ou les colorants de formule (I) conforme à l'invention sont puissantes et permettent d'atteindre une large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière, des lavages, de l'ondulation permanente et de la transpiration.

Dans la formules (I), (II), (III) et (IV) ci-dessus les radicaux alkyle et alcoxy peuvent être linéaires ou ramifiés.
Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.
Les composés de formule (I) conforme à l'invention sont de préférence choisis parmi :
- le chlorure de 1-[2-(2-amino-4-hydroxy-phénylamino)-éthyl]-3-méthyl-3H-imidazol-1-ium ;
- le chlorure de 1-{2-[(3-amino-4-méthylamino-phényl)-méthyl-amino]-éthyl}-3-méthyl-3H-imidazol-1-ium ;
- le chlorure de 1-{2-[(3-amino-4-méthylamino-phényl)-méthyl-amino]-éthyl}-1,4-diméthyl-pipérazin-1-ium ;
- le chlorure de {2-[(3-amino-4-méthylamino-phényl)-méthyl-amino]-éthyl}-triéthyl-ammonium ;
- le chlorure de 1-[2-(2-amino-4-hydroxy-phénylamino)-éthyl]-1,4-diméthylpipérazin-1-ium ;
- le chlorure de (2-{2-amino-4-[bis-(2-hydroxy-éthyl)-amino]-phénylamino}-éthyl)diéthyl-méthyl-ammonium ;
- le dichlorure de 3-(3-{3-[3-(3-méthyl-3H-imidazol-1-ium)-propylamino]-4-aminophénylamino}-propyl)-1-méthyl-3H-imidazol-1-ium ;
- le chlorure de 3-méthyl-1-[2-(2,4,5-trihydroxy-phénylamino)-éthyl]-3H-imidazol-1-ium ;
- le chlorure de 1-[2-(2-amino-5-éthoxy-4-hydroxy-phénylamino)-éthyl]-3-méthyl-3H-imidazol-1-ium ;
- le chlorure de 1-[2-(2-amino-5-hydroxy-4-méthoxy-phénylamino)-éthyl]-3-méthyl-3H-imidazol-1-ium ;
- le dichlorure de 1-(2-{4-amino-5-[2-(diéthyl-méthyl-ammonium)-éthylamino]-2-hydroxy-phénoxy}-éthyl)-3-méthyl-3H-imidazol-1-ium ;
et leurs sels d'addition avec un acide.

Les sels d'addition avec un acide des composés de formule (I) conformes à l'invention sont de préférence choisis parmi les chlorhydrates, les bromhydrates, les sulfates, les citrates, les succinates, les tartrates, les lactates et les acétates.

Les composés de formule (I) conformes à l'invention peuvent être facilement obtenus, selon des méthodes bien connues de l'état de la technique, par réduction des composés nitrés cationiques correspondants.

Cette étape de réduction (obtention d'une amine aromatique primaire) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quaternisation) et terminer par la "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Lorsque la synthèse est terminée, les composés de formule (I) conformes à l'invention peuvent, le cas échéant, être récupérés par des méthodes bien connues de l'état de la technique telles que la cristallisation, la distillation.

Un autre objet de l'invention est l'utilisation des composés de formule (I) conformes à l'invention à titre de précurseurs de colorants d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de précurseur de colorant d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau.
A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 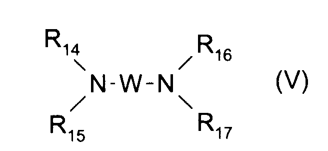 dans laquelle W est un reste propylène éventuellement substitué par un groupement hydroxyle ou un radical alkyle en C₁-C₆ ; R₁₄, R₁₅, R₁₆ et R₁₇, identiques ou différents, représentent un atome d'hydrogène, un radical alkyle en C₁-C₆ ou hydroxyalkyle en C₁-C₆.

La composition tinctoriale conforme à l'invention peut encore contenir, en plus du ou des composés de formule (I) définie ci-dessus, au moins une base d'oxydation qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques non cationiques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)éthanol, le 2-(7-amino pyrazolo-[1,5-a]-pyrimidin-3-ylamino)-éthanol, le 2-[(3-aminopyrazolo[1,5-a]pyrimidin-7-yl)-(2-hydroxy-éthyl)-amino]-éthanol, le 2-[(7-aminopyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques, les pyrazolo-azoles et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'a-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, le 3,6-diméthyl-pyrazolo [3,2-c]-1,2,4-triazole, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre de l'invention (composés de formule (I), bases d'oxydation et coupleurs) sont notamment choisis parmi les chlorhydrates, les bromhydrates, les sulfates, les citrates, les succinates, les tartrates, les lactates et les acétates.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, et notamment lorsque la composition tinctoriale conforme à l'invention renferme une ou plusieurs bases d'oxydation et/ou un ou plusieurs coupleurs, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant présenter un caractère limitatif.

### EXEMPLES DE PREPARATION

### EXEMPLE DE PREPARATION 1 : Synthèse du chlorure de 1-[2-(2-amine-4-hydroxy-phénylamino)-éthyl]-3-méthyl-3H-imidazol-1-ium, dichlorhydrate

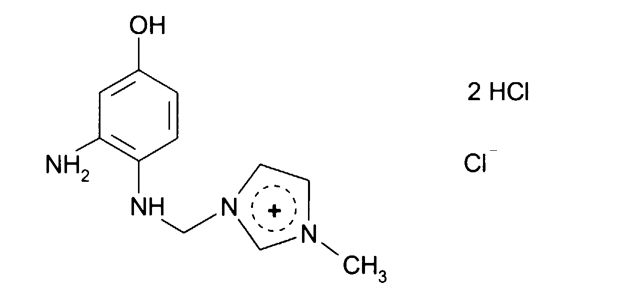

### a) Préparation du 2-(4-benzyloxy-2-nitro-phénylamino)-éthanol

On a fait la suspension de 115 g (0,58 mole) de 4-(2-hydroxy-éthylamino)-3-nitrophénol (RN 65235-31-6) et de 96,2 g (0,696 mole) de carbonate de potassium dans 350 ml de diméthylformamide et chauffé au bain marie bouillant.
On a ajouté goutte à goutte en 20 minutes 73,6 ml (0,638 mole) de chlorure de benzyle et continué le chauffage pendant 3 heures au bain marie bouillant.
On a versé dans 2 litres d'eau glacée, essoré le précipité cristallisé et réempaté dans l'eau.
Après recristallisation de l'éthanol 96° au reflux on a obtenu 145 g de cristaux brun rouge de 2-(4-benzyloxy-2-nitro-phénylamino)-éthanol qui ont fondu à 112°C (Kofler) et dont l'analyse élémentaire calculée pour C₁₅H₁₆N₂O₄ était :

| % | C | H | N | O |
|---|---|---|---|---|
| Calculé | 62,49 | 5,59 | 9,72 | 22,20 |
| Trouvé | 62,45 | 5,61 | 9,60 | 22,35 |

### b) Préparation du (4-benzyloxy-2-nitro-phényl)-(2-chloro-éthyl)-amine

On a fait la solution de 144,5 g (0,5 mole) de 2-(4-benzyloxy-2-nitro-phénylamino)éthanol obtenu ci-dessus à l'étape précédente et de 97 ml de triéthylamine (0,7 mole) dans 750 ml de diméthylformamide et refroidit à O°C.
On a coulé goutte à goutte en 40 minutes 58,0 ml (0,6 mole) de méthanesulfochlorure en maintenant la température entre 0 et 5°C.
On a agité à une température de 0°C pendant 30 minutes supplémentaires.
On a ajouté 63,5 g (1,5 mole) de chlorure de lithium et porté 15 minutes au bain marie bouillant.
On a versé dans 2,5 litres d'eau glacée ; un précipité huileux a cristallisé lentement.
On a essoré, réempaté dans l'eau et séché.
Après recristallisation de l'acétate d'isopropyle au reflux, on a obtenu 102,3 g de cristaux brique de (4-benzyloxy-2-nitro-phényl)-(2-chloro-éthyl)-amine qui ont fondu à 83°C (Kofler) et dont l'analyse élémentaire calculée pour C₁₅H₁₅N₂O₃Cl était :

| % | C | H | N | O | Cl |
|---|---|---|---|---|---|
| Calculé | 58,73 | 4,93 | 9,13 | 15,91 | 11,56 |
| Trouvé | 58,67 | 4,84 | 9,13 | 15,65 | 11,20 |

### c) Préparation du chlorure de 1-[2-(4-benzyloxy-2-nitro-phénylamino)-éthyl]-3-méthyl-3H-imidazol-1-ium, monohydrate.

On a chauffé pendant 12 heures au reflux 30,6 g (0,1 mole) de (4-benzyloxy-2-nitrophényl)-(2-chloro-éthyl)-amine obtenu ci-dessus à l'étape précédente et 24,6 g (0,3 mole) de 1-méthyl-1H-imidazole (RN 616-47-7) dans 60 ml de toluène.
On a refroidit, essoré le précipité cristallisé et réempaté dans l'acétate d'éthyle.
Après recristallisation de l'éthanol absolu au reflux on a obtenu 27,2 g de cristaux rouge orangé de chlorure de 1-[2-(4-benzyloxy-2-nitro-phénylamino)-éthyl]-3-méthyl-3H-imidazol-1-ium, monohydrate qui ont fondu à 145°C (Kofler) et dont l'analyse élémentaire calculée pour C₁₉H₂₁N₄O₃Cl + H₂O était :

| % | C | H | N | O | Cl |
|---|---|---|---|---|---|
| Calculé | 56,09 | 5,70 | 13,77 | 15,73 | 8,71 |
| Trouvé | 57,29 | 5,69 | 13,88 | 15,10 | 8,66 |

### d) Réduction et débenzylation du chlorure de 1-[2-(4-benzyloxy-2-nitro-phénylamino)éthyl]-3-méthyl-3H-imidazol-1-ium, monohydrate

Dans un hydrogénateur on a placé 15,3 g (0,0376 mole) de chlorure de 1-[2-(4-benzyloxy-2-nitro-phénylamino)-éthyl]-3-méthyl-3H-imidazol-1-ium, monohydrate obtenu ci-dessus à l'étape précédente, 5 g de palladium à 5% sur charbon (contenant 50% d'eau), 150 ml d'éthanol à 96° et 150 ml d'eau.
La réduction s'est faite en une ½ heure sous une pression d'hydrogène d'environ 8 bars et à une température qui a progressivement été portée à 45°C.
Après filtration du catalyseur sous azote, on a coulé sur 25 ml d'acide chlorhydrique à 36% et évaporé le filtrat à sec sous pression réduite.
Après recristallisation d'un mélange éthanol/eau au reflux et séchage à 40°C sous vide et sur potasse, on a obtenu 8,0 g de cristaux beiges de chlorure de 1-[2-(2-amino-4-hydroxy-phénylamino)-éthyl]-3-méthyl-3H-imidazol-1-ium, dichlorhydrate, qui ont fondu avec décomposition à 220-222°C (Kofler) et dont l'analyse élémentaire calculée pour C₁₂H₁₉N₄OCl₃ était :

| % | C | H | N | O | Cl |
|---|---|---|---|---|---|
| Calculé | 42,19 | 5,61 | 16,40 | 4,68 | 31,13 |
| Trouvé | 41,95 | 5,69 | 16,13 | 5,63 | 30,62 |

### EXEMPLE DE PREPARATION 2 : Synthèse du chlorure de 1-{2-[(3-amine-4-méthylamino-phényl)-méthyl-amino]-éthyl}-3-méthyl-3H-imidazol-1-ium, dichlorhydrate.

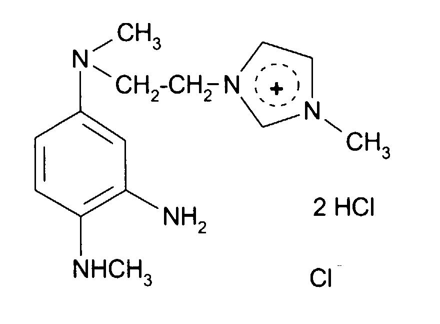

### a) Préparation du chlorure de 3-méthyl-1-{2-[méthyl-(4-méthylamino-3-nitro-phényl)-amino]-éthyl}-3H-imidazol-1-ium, monohydrate

On a réalisé la suspension de 41,4 g (0,17 mole) de N4-(2-chloro-éthyl)-N1,N4-diméthyl-2-nitro-benzène-1,4-diamine (RN14607-54-6) obtenu ci-dessus à l'étape précédente et de 41,8 g (0,51 mole) de 1-méthyl-1H-imidazole (RN 616-47-7) dans 100 ml de toluène.
On a chauffé sous agitation au reflux du toluène pendant 4 heures, essoré bouillant et réempaté deux fois dans l'acétate d'éthyle puis dans l'éthanol absolu.
Après séchage à 40°C sous vide, on a obtenu 37, 8 de cristaux violets de chlorure de 3-méthyl-1-{2-[méthyl-(4-méthylamino-3-nitro-phényl)-amino]-éthyl}-3H-imidazol-1-ium, monohydrate qui ont fondu à 135°C (Kofler) et dont l'analyse élémentaire calculée pour C₁₄H₂₀N₅O₂Cl + H₂O était ;

| % | C | H | N | O | Cl |
|---|---|---|---|---|---|
| Calculé | 48,91 | 6,45 | 20,37 | 13,96 | 10,31 |
| Trouvé | 48,65 | 6,50 | 20,29 | 14,00 | 10,28 |

### b) Réduction du chlorure de 3-méthyl-1-{2-[méthyl-(4-méthylamino-3-nitro-phényl)amino]-éthyl}-3H-imidazol-1-ium, monohydrate

Dans un hydrogénateur, on a placé 19,5 g (0,0567 mole) de chlorure de 3-méthyl-1-{2-[méthyl-(4-méthylamino-3-nitro-phényl)-amino]-éthyl}-3H-imidazol-1-ium, monohydrate obtenu ci-dessus à l'étape précédente, 10 g de palladium à 5% sur charbon (contenant 50% d'eau), 200 ml d'éthanol à 96° et 200 ml d'eau.
La réduction s'est faite en une ½ heure sous une pression d'hydrogène d'environ 9 bars et à une température qui a progressivement été portée à 50°C.
Après filtration du catalyseur sous azote, on a coulé sur 20 ml d'acide chlorhydrique à 36% et évaporé le filtrat à sec sous pression réduite.
Le composé a été repris plusieurs fois dans l'éthanol absolu. Après recristallisation d'un mélange éthanol/eau au reflux et séchage à 40°C sous vide et sur potasse, on a obtenu 8,0 g de cristaux légèrement gris de chlorure de 1-{2-[(3-amino-4-méthylaminophényl)-méthyl-amino]-éthyl}-3-méthyl-3H-imidazol-1-ium, dichlorhydrate, qui ont fondu avec décomposition à 240-242°C (Kofler) et dont le spectre RMN proton était conforme à la structure proposée.

### EXEMPLES D'APPLICATION

### EXEMPLES 1 à 8 DE TEINTURE EN MILIEU ALCALIN

On a préparé les compositions tinctoriales suivantes conformes à l'invention, (teneurs en grammes):

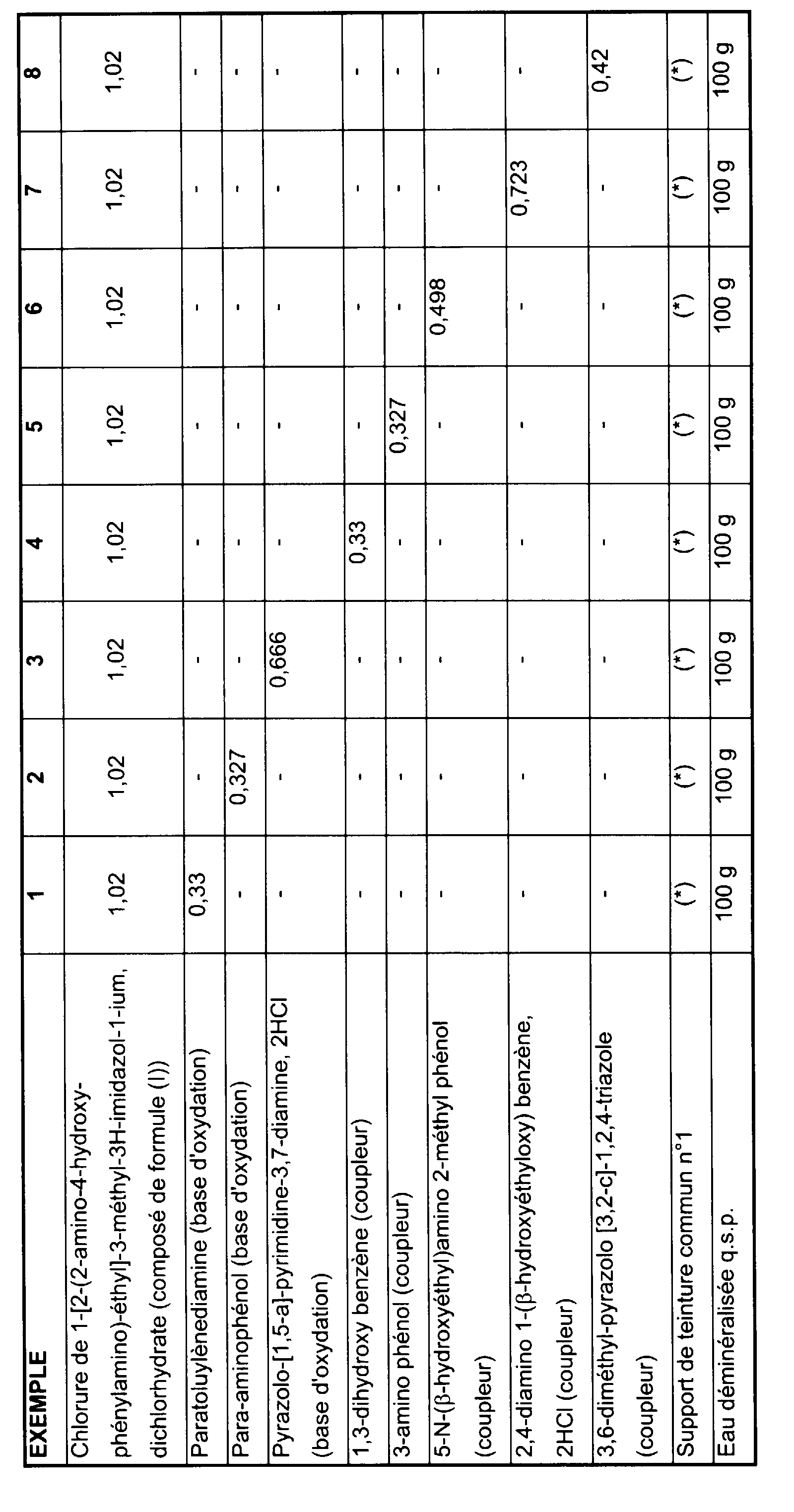

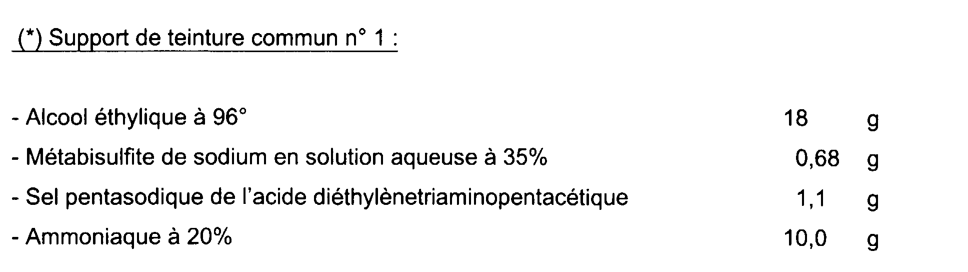

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Blond foncé doré irisé |
| **2** | 10 ± 0,2 | Blond foncé doré cuivré |
| **3** | 10 ± 0,2 | Châtain clair irisé cuivré |
| **4** | 10 ± 0,2 | Blond foncé irisé cuivré |
| **5** | 10 ± 0,2 | Blond foncé irisé cuivré |
| **6** | 10 ± 0,2 | Blond foncé irisé doré |
| **7** | 10 ± 0,2 | Blond foncé cendré mat |
| **8** | 10 ± 0,2 | Blond foncé doré irisé |

### EXEMPLES 9 à 16 DE TEINTURE EN MILIEU ALCALIN

On a préparé les compositions tinctoriales suivantes conformes à l'invention, (teneurs en grammes):

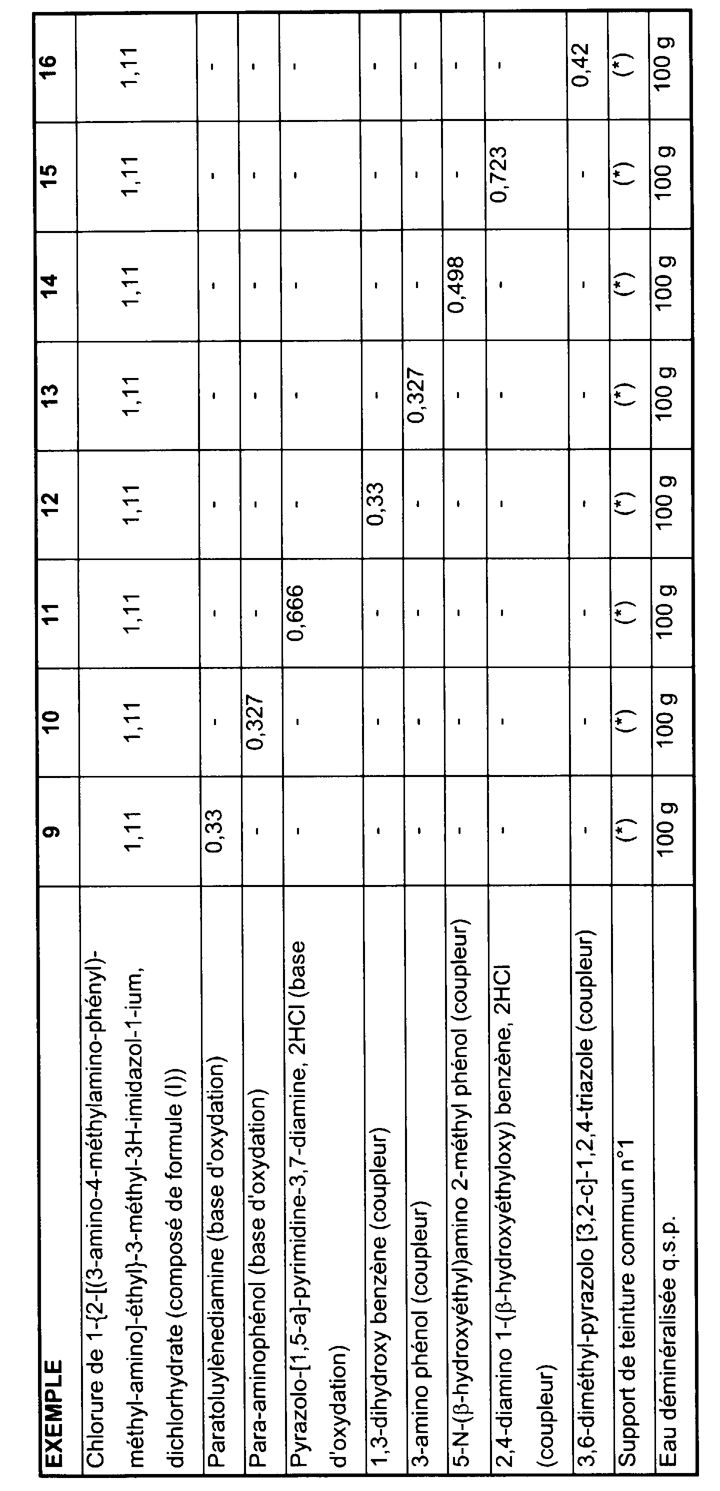

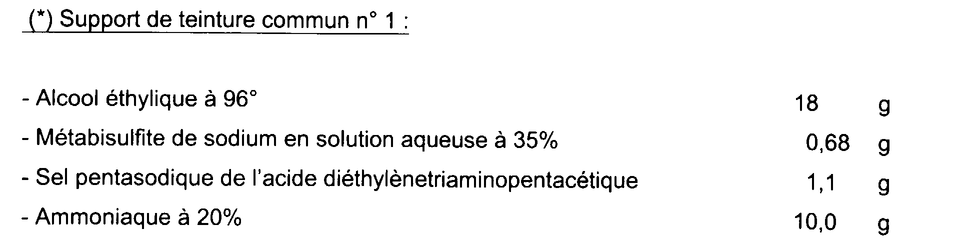

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **9** | 10 ± 0,2 | Blond foncé irisé cuivré |
| **10** | 10 ± 0,2 | Blond cuivré irisé |
| **11** | 10 ± 0,2 | Châtain clair cendré irisé |
| **12** | 10 ± 0,2 | Blond irisé acajou |
| **13** | 10 ± 0,2 | Châtain clair cendré légèrement irisé |
| **14** | 10 ± 0,2 | Châtain clair cendré bleuté |
| **15** | 10 ± 0,2 | Châtain clair cendré bleuté |
| **16** | 10 ± 0,2 | Blond irisé acajou |

## Revendications

1. Composés de formule (I) suivante, et leurs sels d'addition avec un acide : 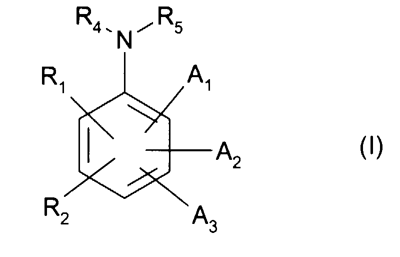 dans laquelle :
• A₁, A₂ et A₃, identiques ou différents, représentent un groupement -NR₇R₈ ou un radical hydroxyle ; un et un seul des groupements A₁, A₂ et A₃ peut en outre représenter un groupement R₃ tel que défini ci-après ;
● • R₁, R₂ et R₃, qui peuvent être identiques ou différents, représentent un atome d'hydrogène ; un atome d'halogène ; un groupement Z tel que défini ci-après ; un radical alkyl(C₁-C₆) carbonyle ; un radical aminoalkyl(C₁-C₆)carbonyle; un radical N-Z-aminoalkyl(C₁-C₆)carbonyle; un radical N-alkyl(C₁-C₆)aminoalkyl(C₁-C₆)carbonyle ; un radical N,N-dialkyl(C₁-C₆)aminoalkyl(C₁-C₆)carbonyle; un radical aminoalkyl(C₁-C₆)carbonylalkyle(C₁-C₆) ; un radical N-Z-aminoalkyl(C₁-C₆)carbonylalkyle(C₁-C₆); un radical N-alkyl(C₁-C₆)aminoalkyl(C₁-C₆)carbonylalkyle(C₁-C₆); un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle; un radical N,N-dialkyl(C₁-C₆)carbamyle; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆) carboxy, carbamyle, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, ou par un groupement Z tel que défini ci-après dans lequel le bras de liaison D contient une contient une fonction cétone directement reliée à l'atome d'azote dudit groupe amino ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle, N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z tels définis ci-après, ou pouvant former ensemble, avec l'atome d'azote auquel ils sont rattachés, un cycle à 5 ou 6 chaînons, carboné, ou contenant un ou plusieurs hétéroatomes ;
● • R₆ désigne un radical alkyle en C₁-C₆ ; un radical monohydroxyalkyle en C₁-C₆ ; un radical polyhydroxyalkyle en C₂-C₆ un groupement Z tel que défini ci-après ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, ou parmi les groupements Z tels que définis ci-après ; ou pouvant former ensemble, avec l'atome d'azote auquel ils sont rattachés, un cycle à 5 ou 6 chaînons, carboné, ou contenant un ou plusieurs hétéroatomes ;
● R₄, R₅, R₇ et R₈, identiques ou différents, représentent un atome d'hydrogène ; un groupement Z tel que défini ci-après dans lequel le bras de liaison D ne contient pas de fonction cétone directement reliée à l'atome d'azote portant les radicaux R₄ et R₅ ; un radical alkyle en C₁-C₆ ; un radical monohydroxyalkyle en C₁-C₆ ; un radical polyhydroxyalkyle en C₂-C₆ un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par un ou plusieurs radicaux hydroxy ; un radical aminoalkyle en C₁-C₆ dont l'alkyle est substitué ou non substitué par un ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle, N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z tels que définis ci-après ; ou pouvant former ensemble, avec l'atome d'azote auquel ils sont rattachés, un cycle à 5 ou 6 chaînons, carboné, ou contenant un ou plusieurs hétéroatomes ;
● Z est choisi parmi les groupements cationiques insaturés de formules (II) et III) suivantes, et les groupements cationiques saturés de formule (IV) suivante : 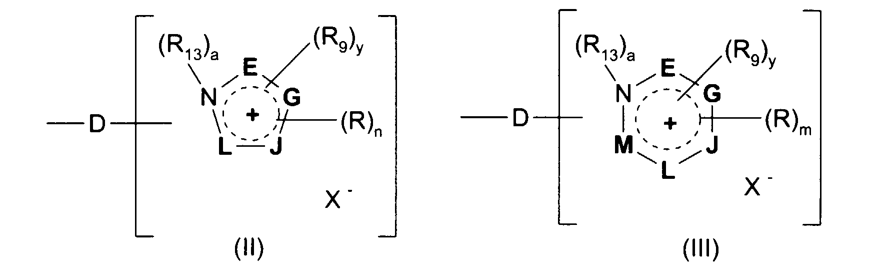 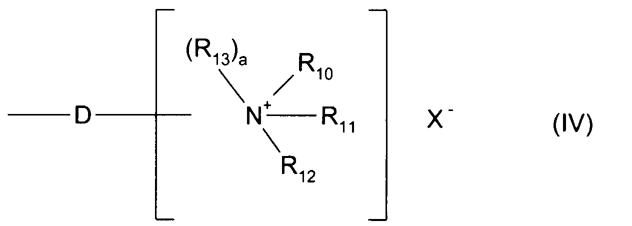 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C2-C6, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₉ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un second groupement Z identique ou différent du premier groupement Z ;
• R₁₀, R₁₁ et R₁₂, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₁₀, R₁₁ et R₁₂ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₁₀, R₁₁ et R₁₂ peut également représenter un second groupement Z, identique ou différent du premier groupement Z ;
• R₁₃ représente un radical alkyle en C₁-C₆ ; un radical monohydroxyalkyle en C₁-C₆ ; un radical polyhydroxyalkyle en C₂-C₆ un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• a et y sont des nombres entiers égaux à 0 ou 1 ; avec les conditions suivantes :
- dans les groupements cationiques insaturés de formule (II);
- lorsque a = 0, le bras de liaison D est rattaché à l'atome d'azote,
- lorsque a = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₉ est porté par l'atome d'azote du cycle insaturé ; ou bien
2) lorsqu'au moins un des sommets E, G, J et L représente un atome d'azote sur lequel le radical R₉ est fixé ;
- dans les groupements cationiques insaturés de formule (III):
- lorsque a = 0, le bras de liaison D est rattaché à l'atome d'azote,
- lorsque a = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₉ est porté par l'atome d'azote du cycle insaturé ;
- dans les groupements cationiques de formule (IV) :
- lorsque a = 0, alors le bras de liaison D est rattaché à l'atome d'azote portant les radicaux R₁₀ à R₁₂,
- lorsque a = 1, alors deux des radicaux R₁₀ à R₁₂ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X⁻ représente un anion monovalent ou divalent ;
étant entendu que :
- le nombre de groupement cationique Z est au moins égal à 1.

2. Composés selon la revendication 1, caractérisés par le fait que les cycles des groupements insaturés Z de formule (II) sont choisis parmi les cycles pyrrolique, imidazolique, pyrazolique, oxazolique, thiazolique et triazolique.

3. Composés selon la revendication 1, caractérisés par le fait que les cycles des groupements insaturés Z de formule (III) sont choisis parmi les cycles pyridinique, pyrimidinique, pyrazinique, oxazinique et triazinique.

4. Composés selon l'une quelconque des revendications précédentes, caractérisés par le fait que deux des radicaux R₁₀, R₁₁ et R₁₂ forment un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C6)sulfonyle.

5. Composés selon l'une quelconque des revendications précédentes, caractérisés par le fait que X⁻ représente un atome d'halogène, un hydroxyde, un hydrogènesulfate, ou un alkyl(C₁-C₆)sulfate.

6. Composés selon l'une quelconque des revendications précédentes, caractérisés par le fait qu'ils sont choisis parmi :
- le chlorure de 1-[2-(2-amino-4-hydroxy-phénylamino)-éthyl]-3-méthyl-3H-imidazol-1-ium ;
- le chlorure de 1-{2-[(3-amino-4-méthylamino-phényl)-méthyl-amino]-éthyl}-3-méthyl-3H-imidazol-1-ium ;
- le chlorure de 1-{2-[(3-amino-4-méthylamino-phényl)-méthyl-amino]-éthyl}-1,4-diméthyl-pipérazin-1-ium ;
- le chlorure de {2-[(3-amino-4-méthylamino-phényl)-méthyl-amino]-éthyl}-triéthyl-ammonium ;
- le chlorure de 1-[2-(2-amino-4-hydroxy-phénylamino)-éthyl]-1,4-diméthyl-pipérazin-1-ium ;
- le chlorure de (2-{2-amino-4-[bis-(2-hydroxy-éthyl)-amino]-phénylamino}-éthyl)-diéthyl-méthyl-ammonium ;
- le dichlorure de 3-(3-{3-[3-(3-méthyl-3H-imidazol-1-ium)-propylamino]-4-amino-phénylamino}-propyl)-1-méthyl-3H-imidazol-1-ium ;
- le chlorure de 3-méthyl-1-[2-(2,4,5-trihydroxy-phénylamino)-éthyl]-3H-imidazol-1-ium ;
- le chlorure de 1-[2-(2-amino-5-éthoxy-4-hydroxy-phénylamino)-éthyl]-3-méthyl-3H-imidazol-1-ium ;
- le chlorure de 1-[2-(2-amino-5-hydroxy-4-méthoxy-phénylamino)-éthyl]-3-méthyl-3H-imidazol-1-ium ;
- le dichlorure de 1-(2-{4-amino-5-[2-(diéthyl-méthyl-ammonium)-éthylamino]-2-hydroxy-phénoxy}-éthyl)-3-méthyl-3H-imidazol-1-ium ;
et leurs sels d'addition avec un acide.

7. Composés selon l'une quelconque des revendications précédentes, caractérisés par le fait que les sels d'addition avec un acide sont choisis parmi les chlorhydrates, les bromhydrates, les sulfates, les citrates, les succinates, les tartrates, les lactates et les acétates.

8. Utilisation des composés de formule (I) tels que définis à l'une quelconque des revendications 1 à 7, à titre de précurseur de colorant d'oxydation pour la teinture d'oxydation des fibres kératiniques.

9. Composition pour la teinture d'oxydation des fibres kératiniques, caractérisée par le fait qu'elle comprend, dans un milieu approprié pour la teinture, au moins un composé de formule (I) tel que défini à l'une quelconque des revendications 1 à 7.

10. Composition selon la revendication 9, caractérisée par le fait que le ou les composés de formule (I) représentent de 0,0005 à 12 % en poids du poids total de la composition tinctoriale.

11. Composition selon la revendication 10, caractérisée par le fait que le ou les composés de formule (I) représentent de 0,005 à 6 % en poids du poids total de la composition tinctoriale.

12. Composition selon l'une quelconque des revendications 9 à 11, caractérisée par le fait qu'elle renferme une ou plusieurs bases d'oxydation choisies parmi les paraphénylènediamines, les bis-phénylalkylènediamines, les para-aminophénols, les ortho-aminophénols et les bases hétérocycliques.

13. Composition selon la revendication 12, caractérisée par le fait que les bases hétérocycliques sont choisies parmi les dérivés pyridiniques, les dérivés pyrimidiniques et les dérivés pyrazoliques.

14. Composition selon la revendication 12 ou 13, caractérisée par le fait que la ou les bases d'oxydation représentent de 0,0005 à 12 % en poids du poids total de la composition tinctoriale.

15. Composition selon l'une quelconque des revendications 9 à 14, caractérisée par le fait qu'elle renferme un ou plusieurs coupleurs choisis parmi les métaphénylènediamines, les méta-aminophénols, les métadiphénols et les coupleurs hétérocycliques, et/ou un ou plusieurs colorants directs.

16. Composition selon la revendication 15, caractérisée par le fait que les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, le 3,6-diméthyl-pyrazolo [3,2-c]-1,2,4-triazole, et leurs sels d'addition avec un acide.

17. Composition selon la revendication 15 ou 16, caractérisée par le fait que le ou les coupleurs représentent de 0,0001 à 10 % en poids environ du poids total de la composition tinctoriale.

18. Composition selon l'une quelconque des revendications 9 à 17, caractérisée par le fait que les sels d'addition avec un acide sont choisis parmi les chlorhydrates, les bromhydrates, les sulfates, les citrates, les succinates, les tartrates, les lactates et les acétates.

19. Procédé de teinture d'oxydation des fibres kératiniques et en particulier des fibres kératiniques humaines telles que les cheveux, caractérisé par le fait qu'on applique sur les fibres au moins une composition tinctoriale telle que définie à l'une quelconque des revendications 9 à 18, pendant un temps suffisant pour développer la coloration désirée, soit à l'air, soit à l'aide d'un agent oxydant.

20. Procédé selon la revendication 19, caractérisé par le fait que la coloration des fibres peut être effectuée sans addition d'un agent oxydant, au seul contact de l'oxygène de l'air.

21. Procédé selon la revendication 19, caractérisé par le fait qu'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

22. Procédé selon la revendication 21, caractérisé par le fait que l'agent oxydant est choisi parmi le peroxyde d'hydrogène, le peroxyde d'urée, les bromates de métaux alcalins, les persels et les enzymes.

23. Procédé selon la revendication 22, caractérisé par le fait que les enzymes sont choisies parmi les peroxydases, les laccases, les tyrosynases et les oxydo-réductases.

24. Procédé selon la revendication 23, caractérisé par le fait que les oxydo-réductases sont choisies parmi les pyranose oxydases, les glucose oxydases, les glycérol oxydases, les lactates oxydases, les pyruvate oxydases, et les uricases.

25. Dispositif à plusieurs compartiments, ou "kit" de teinture à plusieurs compartiments, dont un premier compartiment renferme une composition tinctoriale telle que définie à l'une quelconque des revendications 9 à 18 et un second compartiment renferme une composition oxydante.
